# EUROPEAN PATENT APPLICATION

(11) **EP 3 964 212 A1**
(43) Date of publication of application: **09.03.2022**
(21) Application number: 20799197.7
(22) Date of filing: 30.04.2020
(51) Int. Cl.: A61K 31/454, A61P 37/00, A61P 17/00, A61P 17/06

(54) **COMPOUND FOR TREATING AUTOIMMUNE SKIN DISEASES CAUSED BY INFLAMMATION, AND USE THEREOF**

(30) Priority: 30.04.2019 CN 201910366891
(71) Applicant: Generos Biopharma Ltd., Hangzhou, Zhejiang 310018 (CN)
(72) Inventor: FU, Xin-Yuan, Hangzhou, Zhejiang 310018 (CN); LUFEI, Chengchen, Hangzhou, Zhejiang 310018 (CN); LIU, Xinyu, Hangzhou, Zhejiang 310018 (CN); ZHOU, Yi, Hangzhou, Zhejiang 310018 (CN)
(74) Representative: Williams, Gareth Owen
(86) International application number: PCT/CN2020/088285
(87) International publication number: WO 2020/221347

(57) **Abstract**

Disclosed is a compound, a medicament, and a pharmaceutical composition for treating inflammation induced autoimmune skin diseases, for example psoriasis. The compound is Nib1, a pharmaceutically acceptable salt thereof, a solvate thereof, a solvate of a pharmaceutically acceptable salt thereof, or a crystal form thereof. The compound Nib1 has a chemical formula of C₂₈H₂₉F₂N₃O. It represents a new treatment mechanism and can effectively treat inflammation induced autoimmune skin diseases, for example, non-specific dermatitis such as psoriasis, with less side effects and convenient route of administration, achieving a balance between safety and effectiveness. The compounds can be used for a long time and improve the quality of life of patients. The compounds, medicament, and pharmaceutical compositions can significantly improve clinical symptoms of inflammation induced autoimmune skin diseases, such as non-specific dermatitis such as psoriasis, such as skin rash, skin peeling, skin thickening, and epidermal hyperplasia, improve blood vessel dilation, reduce immune cell infiltration, and etc.

## Description

This application claims the priority of Chinese patent application 201910366891.5, filed April 30, 2019, which is incorporated herein by reference in its entirety.

### Technical Field

The present invention is in the field of medicine and relates to a compound for the treatment of an inflammation induced autoimmune skin disease, a medicament or a pharmaceutical composition comprising the compound, and uses thereof, in particular to a compound with low side effect for treatment of an inflammation induced autoimmune skin disease including psoriasis, medicament or a pharmaceutical composition comprising the compound, and use thereof in the treatment of an inflammation induced autoimmune skin disease including psoriasis.

### Background

Pimozide (C₂₈H₂₉F₂N₃O) is a diphenylbutylpiperidine antipsychotic with similar effect to haloperidol, but acting weaker and longer. It only needs to be taken orally once a day. It shows good efficacy on mania, hallucinations, delusions, apathy withdrawal, and etc. It is especially suitable for patients suffering from chronic Tourette's disease. Pimozide also exhibited calcium antagonism activity. Long-term use of pimozide has not shown systemic risks, nor does it regulate hormones and endocrine system (see, Journal of Neurology, Neurosurgery, and Psychiatry, 1986;49:791-795). The drug has a long history of use, and formal clinical trial records showed that patients had a time of taking the medicine for up to 7 years. If no serious side effects occur, it can be taken for a long time. It was reported that Nib1 was used in dermatology for treating body deformity, metastatic melanoma, trichomoniasis, trigeminal neuralgia and postherpetic neuralgia (see Pimozide in dermatologic practice. Am J Clin Dermatol 2004; 5 (5): 339-349). The skin diseases mentioned were only related to subjective diseases, malignant tumors, parasites or neurological diseases.

Autoimmune diseases are diseases caused by damages induced by immune response of the immune system against the components of the body. Under the influence of certain factors, the body's tissue components or the immune system itself have some abnormalities, causing the immune system to mistake its own components as foreign objects to attack. The immune system then produces antibodies and active lymphocytes against some of the body's own components, damages and destroys its own tissues and organs, leading to the

Inflammation and allergies are one of the main causes of skin diseases. Among them, autoimmune skin diseases caused by autoimmune attacks are a kind of autoimmune diseases, mainly composed of four diseases, *i.e*., psoriasis, usually involved with itching scaly skin; scleroderma, mainly affecting the inner layer of skin tissue and causing thickening of the skin around the hands and feet; hydroa, characteristic of large blisters filled with pus; and alopecia areata, mainly affecting the scalp and can lead to alopecia. Many autoimmune skin diseases are similar to a few other serious skin diseases, especially at the beginning. Autoimmune skin diseases are difficult to diagnose in the early stages because they often look like rashes, allergies, or dry skin spots. These symptoms usually go away on their own or are cured with ointment. A formal diagnosis usually requires blood and other tests, and a confirmed patient often faces a lifetime of treatment.

Psoriasis is a common chronic and recurring skin disease with characteristic skin lesions. The pathogenesis of psoriasis is not yet fully understood, and it is currently believed to be related to a number of factors including genetic factors (it is a polygenic genetic disease that is involved with interaction among a variety of factors such as genetic factors and environmental factors); immune factors (in recent years, it has been widely believed that psoriasis may be an immune or inflammation-mediated disease); infectious factors (studies have confirmed that streptococcal infection, staphylococcus aureus infection, and fungal infection are related to the onset of psoriasis, but it is unclear whether viral infections are related to the onset of psoriasis); endocrine factors (pregnancy can make the skin lesions disappear or reduce, but can also make the skin lesions deteriorate; endocrine diseases such as thyroid disease and diabetes have little effect on the disease); mental factors (patients may experience neuropsychiatric changes, and these changes can deteriorate existing skin lesions); living habits; drugs; and environmental factors.

Studies have found that moisture, infection, drinking, medication, and mental stress are the main risks for inflammation induced autoimmune skin diseases, such as non-specific dermatitis, such as psoriasis. Drugs that may induce or deteriorate inflammation-induced autoimmune skin diseases (such as non-specific dermatitis such as psoriasis) include β1 receptor blockers, non-steroidal anti-inflammatory drugs, lithium salts, antimalarials, tetracyclines, calcium channel blockers, metformin, interferon alpha, and etc. Environmental factors are related to the age of onset, and season and climate play a role in the onset and recurrence of psoriasis.

There are currently many treatment methods for inflammation induced autoimmune skin diseases, such as non-specific dermatitis, such as psoriasis. However, due to the complicated etiology, the curative effect of these methods cannot be determined, and they only improve the symptoms of patients. Traditional therapeutic drugs, especially for patients with autoimmune skin diseases (such as non-specific dermatitis such as psoriasis) caused by moderate to severe inflammation, have many adverse reactions, severe side effects, and no lasting effects, such as hormones such as dexamethasone. Macromolecular biologics also suffer from other safety and drug resistance issues. Due to the above factors or the high price of some drugs, the use of these drugs is restricted.

### Summary

In order to overcome the disadvantages including adverse reactions, severe toxic side effects, lack of safety, drug resistance, and etc. in the treatment of inflammation induced autoimmune skin diseases, for example, non-specific dermatitis such as psoriasis and other diseases, the present invention provides a compound for treating inflammation induced autoimmune skin diseases, for example, non-specific dermatitis such as psoriasis, and a medicament, a pharmaceutical composition and use thereof. The compound, the medicament and the pharmaceutical composition represent a new treatment mechanism and can effectively treat inflammation induced autoimmune skin diseases, for example, non-specific dermatitis such as psoriasis, with less side effects and convenient route of administration, achieving a balance between safety and effectiveness. The compounds can be used for a long time and improve the quality of life of patients. The compounds, medicament, and pharmaceutical compositions can significantly improve clinical symptoms of inflammation induced autoimmune skin diseases, such as non-specific dermatitis such as psoriasis, such as skin rash, skin peeling, skin thickening, and epidermal hyperplasia, improve blood vessel dilation, reduce immune cell infiltration, and etc.

In order to solve the above technical problems, a first aspect of the present invention provides compound Nib1 (Pimozide), a pharmaceutically acceptable salt thereof, a solvate thereof, a solvate of a pharmaceutically acceptable salt thereof, or a crystal form thereof, for use in one or more of the following:
(a) treatment of an inflammation induced autoimmune skin disease;
(b) improvement of skin rash;
(c) improvement of skin peeling;
(d) reduction of skin thickening;
(e) reduction of epidermal hyperplasia;
(f) improvement of blood vessel dilation; and
(g) reduction of immune cell infiltration;
wherein the compound Nib1 has a chemical formula of C₂₈H₂₉F₂N₃O and a structure of

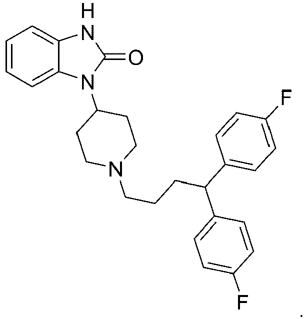

The present inventors discovered that the compound Nib1, a pharmaceutically acceptable salt thereof, a solvate thereof, a solvate of a pharmaceutically acceptable salt thereof, or a crystal form thereof, has a good efficacy for treatment of an inflammation induced autoimmune skin disease, such as non-specific dermatitis such as psoriasis, and less side effects compared with traditional hormone drugs such as dexamethasone, suggesting great potential for preparing a drug to treat an inflammation induced autoimmune skin disease, for example, non-specific dermatitis, such as psoriasis.

In the prior art, hormonal drugs such as dexamethasone have relatively large side effects. Using dexamethasone for more than a few days, common systemic glucocorticoid side effects may occur. These side effects include: stomach upset, increased sensitivity to gastric ulcers; increased appetite, resulting in a significant increase in weight; potential diabetic patients in that glucose intolerance deteriorating the patient's existing diabetes; mental illness, including personality changes, irritability, and mania; long-term treatment of osteoporosis in terms of pathological fractures (such as hip joints); elevated liver enzymes and fatty liver lenticular degeneration (usually reversible); common dependence abstinence syndromes; increased intraocular pressure, including certain types of glaucoma, and cataracts; dermatological diseases including acne, allergic dermatitis, dry scales, ecchymosis, erythema, hard-healing wounds, increased sweating, rash, dermatoglyph that affects the skin reaction test (skin test) results; allergic reactions (although rare) including angioedema (very unlikely, because dexamethasone is given to prevent allergic reactions) and so on.

Long-term use of dexamethasone can damage the endocrine system, leading to atrophy of multiple glands, circulatory failure, and even paralysis of the lower body or even quadriplegia. It is caused by endocrine disorders that cause heart and kidney damage. This type of drug can only be used as an emergency and should not be used as a long-term anti-inflammatory drug. Generally, the compound Nib1 in disclosed herein has no systemic risk after long-term use, and it is not a regulatory hormone of endocrine system.

Preferably, the inflammation induced autoimmune skin disease is non-specific dermatitis, such as psoriasis, scleroderma, hydroa, and/or alopecia areata.

In order to solve the above technical problems, a second aspect of the present invention provides use of compound Nib1, a pharmaceutically acceptable salt thereof, a solvate thereof, a solvate of a pharmaceutically acceptable salt thereof, or a crystal form thereof, in the preparation of a medicament for treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration;
wherein the compound Nib 1 has a structure of

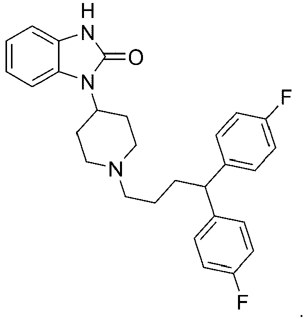

Preferably, the inflammation induced autoimmune skin disease is non-specific dermatitis, such as psoriasis, scleroderma, hydroa, and/or alopecia areata.

In order to solve the above technical problems, a third aspect of the present invention provides a pharmaceutical composition comprising compound Nib1, a pharmaceutically acceptable salt thereof, a solvate thereof, a solvate of a pharmaceutically acceptable salt thereof, or a crystal form thereof, for one or more of the following:
(a) treatment of an inflammation induced autoimmune skin disease;
(b) improvement of skin rash;
(c) improvement of skin peeling;
(d) reduction of skin thickening;
(e) reduction of epidermal hyperplasia;
(f) improvement of blood vessel dilation; and
(g) reduction of immune cell infiltration;
wherein the compound Nib 1 has a structure of

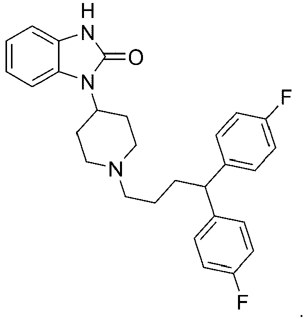

Preferably, the inflammation induced autoimmune skin disease is non-specific dermatitis, such as psoriasis, scleroderma, hydroa, and/or alopecia areata.

Preferably, the pharmaceutical composition further comprises a pharmaceutically acceptable carrier, an excipient and/or a solvent.

Preferably, the pharmaceutical composition further comprises a further drug, wherein, the further drug may be a drug for treating an inflammation induced autoimmune skin disease; and/or the further drug may also be a drug for improvement of skin rash, improvement of skin peeling, reduction of skin thickening, reduction of epidermal hyperplasia, improvement of blood vessel dilation and/or reduction of immune cell infiltration.

Preferably, the pharmaceutical composition is in the form of a preparation for internal or external use.

Preferably, the pharmaceutical composition is in the form of an oral preparation or a preparation for external application.

When the pharmaceutical composition is in the form of an oral preparation, the compound Nib1, a pharmaceutically acceptable salt thereof, a solvate thereof, a solvate of a pharmaceutically acceptable salt thereof, or a crystal form thereof, in the oral preparation, is administered at preferably 0.1-1.098 mg/kg per time, once a day or as needed.

In the present disclosure, the dosage is generally the dosage for human body, which is calculated according to the dosage of the animal experiment of the present invention, and specifically is the mouse dosage divided by the conversion factor 9.1. However, those skilled in the art should understand that the dosages within a generally accepted error range in the art should be also within the scope of the present invention, for example, the difference is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, and etc.

When the pharmaceutical composition is in the form of a preparation for external application, the compound Nib1, a pharmaceutically acceptable salt thereof, a solvate thereof, a solvate of a pharmaceutically acceptable salt thereof, or a crystal form thereof, in the preparation is administered at a dosage suitable to cover the lesion area, for example, 5-15 mg/cm² each time, at least once a day or as needed. In a preferred embodiment of the present invention, 62.5 mg of the preparation for external application is applied daily on a site with an area of 2×3 cm for seven consecutive days.

In a preferred embodiment of the present invention, the pharmaceutical composition is in the form of a preparation for external application, and the preparation for external application comprises, by weight, 0.2-0.3 parts of the compound Nib1, a pharmaceutically acceptable salt thereof, a solvate thereof, a solvate of a pharmaceutically acceptable salt thereof, or a crystal form thereof, 9-11 parts of an oil phase, 1.3-2.6 parts of an emulsifier, 6.5-8.5 parts of a solvent and 25-35 parts of water.

Preferably, the compound Nib1, a pharmaceutically acceptable salt thereof, a solvate thereof, a solvate of a pharmaceutically acceptable salt thereof, or a crystal form thereof is 0.25 parts by weight.

Preferably, the oil phase includes one or more oil phases, preferably two oil phases.

Preferably, the oil phase includes a solidified oil phase and an ordinary oil phase. The solidified oil phase is preferably 5-6 parts, such as 5.5 parts, and the ordinary oil phase is preferably 4-5 parts, such as 4.5 parts. The solidified oil phase is preferably cetostearyl alcohol, and the ordinary oil phase is preferably medium chain triglycerides.

In the present invention, the solidified oil phase and the ordinary oil phase are technical terms in the field of external application preparations, which have the usual meanings understood by those skilled in the art. Generally, the ordinary oil phase is the oil phase conventionally used in the art, excluding the solidified oil phase, and the solidified oil phase generally refers to the oil phase that can solidify and shape in the art.

Preferably, the emulsifier includes one or more emulsifiers, and preferably includes Tween 60 and Span 80, wherein the weight of Tween 60 is preferably 1-2 parts, such as 1.5 parts, and the weight of Span 80 is preferably 0.3-0.6 parts, for example 0.35, 0.4 or 0.5 parts.

Preferably, the solvent includes one or more solvents, preferably including dimethyl sulfoxide and benzyl alcohol, wherein the weight of the dimethyl sulfoxide is preferably 2.5-3.5 parts, such as 3 parts, and the weight of benzyl alcohol is preferably 4-5 parts, for example 4.5 parts.

Preferably, the weight of the water is preferably 30 parts or 30.25 parts.

Preferably, the preparation for external application further includes acetic acid, and the weight of the acetic acid is preferably 0.3-0.5 parts, more preferably 0.4 parts.

In a preferred embodiment of the present invention, the preparation for external application comprises by weight 0.25 parts of the compound Nib 1, a pharmaceutically acceptable salt thereof, a solvate thereof, a solvate of a pharmaceutically acceptable salt thereof, or a crystal form thereof, 0.4 parts of acetic acid, 5.5 parts of cetostearyl alcohol, 4.5 parts of medium chain triglycerides, 1.5 parts of Tween 60, 0.35 parts of Span 80, 3 parts of dimethyl sulfoxide, 4.5 parts of benzyl alcohol, and 30 parts of water.

In a preferred embodiment of the present invention, the preparation for external application comprises by weight 0.25 parts of the compound Nib1, a pharmaceutically acceptable salt thereof, a solvate thereof, a solvate of a pharmaceutically acceptable salt thereof, or a crystal form thereof, 5.5 parts of cetostearyl alcohol, 4.5 parts of medium chain triglycerides, 1.5 parts of Tween 60, 0.5 parts of Span 80, 3 parts of dimethyl sulfoxide, 4.5 parts of benzyl alcohol, and 30.25 parts of water.

In the present invention, the cetostearyl alcohol is cetostearyl alcohol commonly used in the field. In a preferred embodiment of the present invention, the cetostearyl alcohol used was purchased from Hunan Erkang Pharmaceutical Co., Ltd. (catalog # F20170000297).

Preferably, the preparation for external application is prepared by mixing the compound Nib1, a pharmaceutically acceptable salt thereof, a solvate thereof, a solvate of a pharmaceutically acceptable salt thereof, or a crystal form thereof, the oil phase, the emulsifier, the solvent, and water. Generally, it is formulated into a cream. In the present invention, the ointment can be prepared by routine methods in the field, with or without changes, as long as it can achieve a transdermal effect and can be used externally.

More preferably, the preparation for external application can be prepared by first dissolving the compound Nib1, a pharmaceutically acceptable salt thereof, a solvate thereof, a solvate of a pharmaceutically acceptable salt thereof, or a crystal form thereof, in the solvent, followed by adding the oil phase and the emulsifier, and finally adding water.

Even more preferably, the temperature of the dissolution is 65-75°C.

Even more preferably, the dissolution time is 4-6 min.

Even more preferably, after adding the oil phase and the emulsifier, it is placed in an environment with a temperature of preferably 65-75°C for preferably 10-20 min.

Even more preferably, the temperature of the water is 65-75°C.

Even more preferably, after adding the water, the mixture is stirred, preferably until it is cooled to form a cream.

In a preferred embodiment of the present invention, the preparation for external application is prepared by a method comprising the following steps:
(1) weighing Nib1 and adding it in a container, then adding acetic acid to make the acetic acid just over the Nib1 API, and the whole white solid turns into transparent crystals, followed by adding a mixed solvents consisting of dimethyl sulfoxide and benzyl alcohol, stirring at room temperature to dissolve a part of the transparent crystal, and then placing it in a water bath at 65-75°C for 4-6 minutes to dissolve Nib1;
(2) adding medium-chain triglycerides to the solution obtained in step (1); adding cetostearyl alcohol and emulsifier, and placing in a water bath at 65-75°C for 10-20 minutes to obtain a transparent oil phase; and
(3) adding water at the same temperature to the transparent oil phase, taking it out and stirring until it cools to become a cream.

In a preferred embodiment of the present invention, the preparation for external application is prepared by a method comprising the following steps:
(1) weighing Nib1 and adding it to a mixed solvent composed of dimethyl sulfoxide and benzyl alcohol, shaking and stirring, and then placing it in a water bath at 65-75°C for 15-25 minutes to obtain an oil phase solution;
(2) adding medium-chain triglycerides, cetostearyl alcohol, Tween 60 and Span 80 to the oil phase solution, and then heating and melting in a water bath at 65-75°C to obtain a transparent oil phase solution; and
(3) adding water at the same temperature to the transparent oil phase solution, stirring continuously, taking it out and stirring until it cools to become a cream.

In order to solve the above technical problems, a fourth aspect of the present invention provides use of compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention, for treatment of an inflammation induced autoimmune skin disease, or use of compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention, for improvement of skin rash, improvement of skin peeling, reduction of skin thickening, reduction of epidermal hyperplasia, improvement of blood vessel dilation and/or reduction of immune cell infiltration.

Preferably, the inflammation induced autoimmune skin disease is non-specific dermatitis, such as psoriasis, scleroderma, hydroa, and/or alopecia areata.

In order to solve the above technical problems, a fifth aspect of the present invention provides use of compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention, in the preparation of a medicament for treatment of an inflammation induced autoimmune skin disease, or for improvement of skin rash, improvement of skin peeling, reduction of skin thickening, reduction of epidermal hyperplasia, improvement of blood vessel dilation and/or reduction of immune cell infiltration.

Preferably, the inflammation induced autoimmune skin disease is non-specific dermatitis, such as psoriasis, scleroderma, hydroa, and/or alopecia areata.

Preferably, the medicament further comprises a pharmaceutically acceptable carrier, an excipient and/or a solvent.

Preferably, the medicament is in the form of a pharmaceutical composition which preferably further comprises a further drug for treating an inflammation induced autoimmune skin disease; and/or the pharmaceutical composition preferably comprises a further drug for improvement of skin rash, improvement of skin peeling, reduction of skin thickening, reduction of epidermal hyperplasia, improvement of blood vessel dilation and/or reduction of immune cell infiltration.

In order to solve the above technical problems, a sixth aspect of the present invention provides a method for treatment of an inflammation induced autoimmune skin disease for example in a subject in need thereof, and/or a method for improvement of skin rash for example in a subject in need thereof, improvement of skin peeling for example in a subject in need thereof, reduction of skin thickening for example in a subject in need thereof, reduction of epidermal hyperplasia for example in a subject in need thereof, improvement of blood vessel dilation for example in a subject in need thereof and/or reduction of immune cell infiltration for example in a subject in need thereof, comprising using compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention for the treatment, or administering to the subject a therapeutically effective amount of compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention.

Preferably, the inflammation induced autoimmune skin disease is non-specific dermatitis, such as psoriasis, scleroderma, hydroa, and/or alopecia areata.

The present invention further provides a method for treating a subject in need of administering a medicament for treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration, comprising administering to the subject a therapeutically effective amount of compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention.

In order to solve the above technical problems, a seventh aspect of the present invention provides a preparation for external application, comprising, by weight, 0.2-0.3 parts of compound Nib1, a pharmaceutically acceptable salt thereof, a solvate thereof, a solvate of a pharmaceutically acceptable salt thereof, or a crystal form thereof, 9-11 parts of oil phase, 1.3-2.6 parts of emulsifier, 6.5-8.5 parts of solvent, and 25-35 parts of water.

Preferably, the preparation for external application is useful for one or more of the following: (a) treatment of an inflammation induced autoimmune skin disease; (b) improvement of skin rash; (c) improvement of skin peeling; (d) reduction of skin thickening; (e) reduction of epidermal hyperplasia; (f) improvement of blood vessel dilation; and (g) reduction of immune cell infiltration.

Preferably, the compound Nib1, a pharmaceutically acceptable salt thereof, a solvate thereof, a solvate of a pharmaceutically acceptable salt thereof, or a crystal form thereof is 0.25 parts by weight.

Preferably, the oil phase includes one or more oil phases, preferably two oil phases.

Preferably, the oil phase includes a solidified oil phase and an ordinary oil phase. The solidified oil phase is preferably 5-6 parts, such as 5.5 parts, and the ordinary oil phase is preferably 4-5 parts, such as 4.5 parts. The solidified oil phase is preferably cetostearyl alcohol, and the ordinary oil phase is preferably medium chain triglycerides.

Preferably, the emulsifier includes one or more emulsifiers, and preferably includes Tween 60 and Span 80, wherein the weight of Tween 60 is preferably 1-2 parts, such as 1.5 parts, and the weight of Span 80 is preferably 0.3-0.6 parts, for example 0.35, 0.4 or 0.5 parts.

Preferably, the solvent includes one or more solvents, preferably including dimethyl sulfoxide and benzyl alcohol, wherein the weight of the dimethyl sulfoxide is preferably 2.5-3.5 parts, such as 3 parts, and the weight of benzyl alcohol is preferably 4-5 parts, for example 4.5 parts.

Preferably, the weight of the water is preferably 30 parts or 30.25 parts.

Preferably, the preparation for external application further includes acetic acid, and the weight of the acetic acid is preferably 0.3-0.5 parts, more preferably 0.4 parts.

More preferably, the preparation for external application comprises by weight 0.25 parts of the compound Nib1, a pharmaceutically acceptable salt thereof, a solvate thereof, a solvate of a pharmaceutically acceptable salt thereof, or a crystal form thereof, 0.4 parts of acetic acid, 5.5 parts of cetostearyl alcohol, 4.5 parts of medium chain triglycerides, 1.5 parts of Tween 60, 0.35 parts of Span 80, 3 parts of dimethyl sulfoxide, 4.5 parts of benzyl alcohol, and 30 parts of water.

More preferably, the preparation for external application comprises by weight 0.25 parts of the compound Nib1, a pharmaceutically acceptable salt thereof, a solvate thereof, a solvate of a pharmaceutically acceptable salt thereof, or a crystal form thereof, 5.5 parts of cetostearyl alcohol, 4.5 parts of medium chain triglycerides, 1.5 parts of Tween 60, 0.5 parts of Span 80, 3 parts of dimethyl sulfoxide, 4.5 parts of benzyl alcohol, and 30.25 parts of water.

Preferably, the preparation for external application is prepared by mixing the compound Nib1, a pharmaceutically acceptable salt thereof, a solvate thereof, a solvate of a pharmaceutically acceptable salt thereof, or a crystal form thereof, the oil phase, the emulsifier, the solvent, and water. Generally, it is formulated into a cream. In the present invention, the ointment can be prepared by routine methods in the field, with or without changes, as long as it can achieve a transdermal effect and can be used externally.

More preferably, the preparation for external application can be prepared by first dissolving the compound Nib1, a pharmaceutically acceptable salt thereof, a solvate thereof, a solvate of a pharmaceutically acceptable salt thereof, or a crystal form thereof, in the solvent, followed by adding the oil phase and the emulsifier, and finally adding water.

Even more preferably, the temperature of the dissolution is 65-75°C.

Even more preferably, the dissolution time is 4-6 min.

Even more preferably, after adding the oil phase and the emulsifier, it is placed in an environment with a temperature of preferably 65-75°C for preferably 10-20 min.

Even more preferably, the temperature of the water is 65-75°C.

Even more preferably, after adding the water, the mixture is stirred, preferably until it is cooled to form a cream.

In a preferred embodiment of the present invention, the preparation for external application is prepared by a method comprising the following steps:
(1) weighing Nib1 and adding it in a container, then adding acetic acid to make the acetic acid just over the Nib1 API, and the whole white solid turns into transparent crystals, followed by adding a mixed solvents consisting of dimethyl sulfoxide and benzyl alcohol, stirring at room temperature to dissolve a part of the transparent crystal, and then placing it in a water bath at 65-75°C for 4-6 minutes to dissolve Nib1;
(2) adding medium-chain triglycerides to the solution obtained in step (1); adding cetostearyl alcohol and emulsifier, and placing in a water bath at 65-75°C for 10-20 minutes to obtain a transparent oil phase; and
(3) adding water at the same temperature to the transparent oil phase, taking it out and stirring until it cools to become a cream.

In a preferred embodiment of the present invention, the preparation for external application is prepared by a method comprising the following steps:
(1) weighing Nib1 and adding it to a mixed solvent composed of dimethyl sulfoxide and benzyl alcohol, shaking and stirring, and then placing it in a water bath at 65-75°C for 15-25 minutes to obtain an oil phase solution;
(2) adding medium-chain triglycerides, cetostearyl alcohol, Tween 60 and Span 80 to the oil phase solution, and then heating and melting in a water bath at 65-75°C to obtain a transparent oil phase solution; and
(3) adding water at the same temperature to the transparent oil phase solution, stirring continuously, taking it out and stirring until it cools to become a cream.

In order to solve the above technical problems, the present invention also provides a kit, comprising a package A which is compound Nib1, a pharmaceutically acceptable salt thereof, a solvate thereof, a solvate of a pharmaceutically acceptable salt thereof, or a crystal form thereof, as provided in the first or second aspect of the present invention, or the pharmaceutical composition as provided in the third aspect of the present invention, and a package B which is a further drug for the treatment of inflammation induced autoimmune skin diseases, or for improvement of skin rash, improvement of skin peeling, reduction of skin thickening, reduction of epidermal hyperplasia, improvement of blood vessel dilation and/or reduction of immune cell infiltration. The packages A and B can be used simultaneously, or sequentially in the sequence of A to B or B to A as determined as needed.

In order to solve the above technical problems, the present invention also provides a medicament with low side effects for treatment of psoriasis, comprising compound Nib 1 and/or a pharmaceutically acceptable salt thereof, wherein compound Nib1 has chemical formula of C₂₈H₂₉F₂N₃O and a structure of

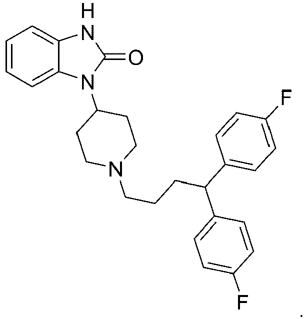

Preferably, the medicament for treatment of psoriasis further comprises a pharmaceutically acceptable carrier, an excipient and/or a solvent.

Preferably, the medicament for treatment of psoriasis is in the form of a preparation for internal or external use.

Preferably, the medicament for treatment of psoriasis is in the form of an oral preparation or a preparation for external application.

Preferably, the compound Nib1 in the oral preparation is administered at 0.1-1.098 mg/kg per time, once a day.

Preferably, the compound Nib1 in the preparation for external application is administered at a dosage suitable to cover the lesion area, for example, 5-15 mg/cm² each time, at least once a day or as needed.

Preferably, the preparation for external application comprises, by weight, 0.2-0.3 parts of Nib1 API, 0.3-0.5 parts of acetic acid, 5-6 parts of oil phase A, 4-5 parts of oil phase B, 1-2 parts of emulsifier A, 0.3-0.4 parts of emulsifier B, 2.5-3.5 parts of solvent A, 4-5 parts of solvent B, and 25-35 parts of water.

Also preferably, the preparation for external application comprises, by weight, 0.2-0.3 parts of Nib1 API, 5-6 parts of oil phase A, 4-5 parts of oil phase B, 1-2 parts of emulsifier A, 0.4-0.6 parts of emulsifier B, 2.5-3.5 parts of solvent A, 4-5 parts of solvent B, and 25-35 parts of water.

More preferably, the oil phase A is cetostearyl alcohol, the oil phase B is medium chain triglycerides, the emulsifier A is Tween 60, the emulsifier B is Span 80, the solvent A is dimethyl sulfoxide, and the solvent B is benzyl alcohol.

Further preferably, the preparations for external application are prepared respectively by the following methods.

### Method A (using acetic acid)

(1) weighing Nib1 and adding it in a container, then adding acetic acid to make the acetic acid just over the Nib1 API, and the whole white solid turns into transparent crystals, followed by adding a mixed solvents consisting of dimethyl sulfoxide and benzyl alcohol, stirring at room temperature to dissolve a part of the transparent crystal, and then placing it in a water bath at 65-75°C for 4-6 minutes to dissolve Nib1;
(2) adding medium-chain triglycerides to the solution obtained in step (1); adding cetostearyl alcohol and emulsifier, and placing in a water bath at 65-75°C for 10-20 minutes to obtain a transparent oil phase; and
(3) adding water at the same temperature to the transparent oil phase, taking it out and stirring until it cools to become a cream.

### Method B (acetic acid free)

(1) weighing Nib1 and adding it to a mixed solvent composed of dimethyl sulfoxide and benzyl alcohol, shaking and stirring, and then placing it in a water bath at 65-75°C for 15-25 minutes to obtain an oil phase solution;
(2) adding medium-chain triglycerides, cetostearyl alcohol, Tween 60 and Span 80 to the oil phase solution, and then heating and melting in a water bath at 65-75°C to obtain a transparent oil phase solution; and
(3) adding water at the same temperature to the transparent oil phase solution, stirring continuously, taking it out and stirring until it cools to become a cream.

### Definition of Terminologies

The term "pharmaceutically acceptable" means that salts, solvents, excipients, etc. are generally non-toxic, safe, and suitable for use by patients. The "patient" is preferably a mammal, more preferably a human.

The term "pharmaceutically acceptable salt" refers to a salt prepared from a compound of the present invention, a medicament or a pharmaceutical composition containing the compound, with a relatively non-toxic, pharmaceutically acceptable acid or base. When the compound of the present invention, a medicament or a pharmaceutical composition containing the compound, contains a relatively acidic functional group, a base addition salt can be obtained by contacting neutral form of the compound with a sufficient amount of a pharmaceutically acceptable base in a pure solution or a suitable inert solvent. Pharmaceutically acceptable base addition salts include, but are not limited to, lithium salt, sodium salt, potassium salt, calcium salt, aluminum salt, magnesium salt, zinc salt, bismuth salt, ammonium salt, and diethanolamine salt. When the drug of the present invention contains a relatively basic functional group, an acid addition salt can be obtained by contacting the neutral form of the drug with a sufficient amount of a pharmaceutically acceptable acid in a pure solution or a suitable inert solvent. The pharmaceutically acceptable acids include inorganic acids, including but are not limited to, hydrochloric acid, hydrobromic acid, hydroiodic acid, nitric acid, carbonic acid, phosphoric acid, phosphorous acid, sulfuric acid, and the like. The pharmaceutically acceptable acids include organic acids, including but not limited to, acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid , tannic acid, pantothenic acid, hydrogen tartrate, ascorbic acid, gentisic acid, fumaric acid, gluconic acid, sugar acid, formic acid, ethanesulfonic acid, pamoic acid (i.e., 4, 4'-methylene-bis(3-hydroxy-2-naphthoic acid)), amino acids (such as glutamic acid, arginine), and etc. When the drug of the present invention contains relatively acidic and relatively basic functional groups, it can be converted into a base addition salt or an acid addition salt. For details, see Berge et al., "Pharmaceutical Salts", Journal of Pharmaceutical Science 66: 1-19 (1977) or Handbook of Pharmaceutical Salts: Properties, Selection, and Use (P. Heinrich Stahl and Camille G. Wermuth, ed., Wiley-VCH, 2002).

The "more" in the term "one or more" may refer to 2, 3, 4, 5, 6, 7, 8, 9, or greater.

The compound, the medicament or the pharmaceutical composition of the present invention can be administered in a unit dosage form, and the route of administration can be parenteral or non-parenteral, such as oral, topical, intravenous injection, intramuscular injection, subcutaneous injection, nasal cavity, oral mucosa, eyes, lungs, respiratory tract, skin, vagina, rectum, etc., preferably by oral or external application.

The dosage form for administration may be a liquid, a solid or a semi-solid dosage form. Liquid dosage forms can be solutions (including true solutions and colloidal solutions), emulsions (including o/w type, w/o type and multiple emulsion), suspensions, injections (including water injections, powder injections and infusions), eye drops, lotion and liniment. Solid dosage forms can be tablets (including ordinary tablets, enteric-coated tablets, buccal tablets, dispersible tablets, chewable tablets, effervescent tablets, orally disintegrating tablets), capsules (including hard capsules, soft capsules, enteric-coated capsules), granules, powders, pellets, dripping pills, suppositories, films, patches, aerosol, powder inhalation, sprays, and etc. Semi-solid dosage forms can be ointments, gels, pastes, and etc.

The medicament or pharmaceutical composition of the present invention can be made into ordinary preparations, and can also be made into sustained-release preparations, controlled-release preparations, targeted preparations and various particle delivery systems.

"Pharmaceutical composition" refers to mixing one or more of the compounds of the present invention or their pharmaceutically acceptable salts, solvates, hydrates or prodrugs with other chemical ingredients, such as pharmaceutically acceptable carriers. The purpose of the pharmaceutical composition is to facilitate the process of administration to animals.

"Pharmaceutically acceptable carrier" refers to an inactive ingredient in a pharmaceutical composition that does not cause significant irritation to the organism and does not interfere with the biological activity and properties of the administered compound, such as but not limited to, calcium carbonate, calcium phosphate, various sugars (such as lactose, mannitol, etc.), starch, cyclodextrin, stearic acid, cellulose, carbonate, acrylic acid polymer or methacrylic acid polymer, gels, water, polyethylene glycol, propylene glycol, ethylene glycol, EZ oil or hydrogenated EZ oil or polyethoxylated hydrogenated EZ oil, sesame oil, corn oil, peanut oil, and etc.

The pharmaceutical composition, in addition to a pharmaceutically acceptable carrier, may also include auxiliary agents commonly used in pharmacology, such as antibacterial agents, antifungal agents, antimicrobial agents, stabilizing agents, colorants, solubilizers, thickeners, surfactants, complexing agents, proteins, amino acids, fats, carbohydrates, vitamins, minerals, trace elements, sweeteners, pigments, flavors or their combination.

The term "treatment" refers to therapeutic therapy. When referring to a specific disease, treatment refers to (1) alleviating one or more biological manifestations of the disease or disorder, (2) interfering with (a) one or more points in the biological cascade causing or inducing the disease, or (b) ) one or more biological manifestations of the disorder, (3) improvement of one or more symptoms, effects or side effects related to the disorder, or one or more symptoms, effects or side effects related to its treatment, or (4) slowing down the development or one or more biological manifestations of the disease.

The term "solvate" refers to a substance formed by combining the compound of the present invention with a stoichiometric or non-stoichiometric solvent. The solvent molecules in the solvate can exist in an ordered or non-ordered arrangement. The solvents include but are not limited to water, methanol, ethanol and the like.

The "pharmaceutically acceptable salt" and "solvate" in the term "a solvate of a pharmaceutically acceptable salt" are defined as above, and the latter refers to a substance formed by combining a salt prepared from a compound of the present invention with a relatively non-toxic, pharmaceutically acceptable acid or base, with a stoichiometric or non-stoichiometric solvent. The "solvate of a pharmaceutically acceptable salt" includes, but is not limited to, the hydrochloric acid monohydrate of the compound of the present invention.

The terms "compound", "pharmaceutically acceptable salt", "solvate" and "solvate of pharmaceutically acceptable salt" may exist in crystalline or amorphous forms. The term "crystal form" means that the ions or molecules are arranged in strictly periodical manner in a three-dimensional space in a certain way, and occur periodically at intervals. Due to the above-mentioned periodic arrangement, there may be multiple crystal forms, i.e., polymorphism. The term "amorphous" means that the ions or molecules present in a disorderly distribution state, that is, there is no periodic arrangement between the ions and molecules.

In the present invention, the "including", "containing" or "comprising" may mean that in addition to the ingredients listed, there are other ingredients; it may also mean "consisting of', that is, only including the ingredients listed and without including other ingredients.

The present invention is advantageous over the prior art in that it represents a new treatment mechanism and can effectively treat inflammation induced autoimmune skin diseases, for example, non-specific dermatitis such as psoriasis, with less side effects and convenient route of administration, achieving a balance between safety and effectiveness. The compounds can be used for a long time and improve the quality of life of patients. The compounds, medicament, and pharmaceutical compositions can significantly improve clinical symptoms of inflammation induced autoimmune skin diseases, such as non-specific dermatitis such as psoriasis, such as skin rash, skin peeling, skin thickening, and epidermal hyperplasia, improve blood vessel dilation, reduce immune cell infiltration, and etc.

### Brief Description of the Drawings

Figure 1 shows the changes of the animal body weight in Example 1 during the experiment.
Figure 2 shows the clinical scoring data of the rash of the psoriasis model in Example 1.
Figure 3 is the clinical score data of the peeling symptoms of the psoriasis model in Example 1.
Figure 4 is the clinical score data of the skin thickening symptoms of the psoriasis model in Example 1.
Figure 5 is the data of the total clinical score of the psoriasis model in Example 1.
Fig. 6 is the comparison result of the area under the clinical total score curve of the psoriasis model in Example 1.
Figure 7 shows the staining results of the skin pathological section of the normal group in Example 1 (animal number G1-309, 100x magnification).
Figure 8 shows the staining results of the skin pathological section of the normal group in Example 1 (animal number G1-316, 100x magnification).
Figure 9 shows the staining results of the skin pathological section of the normal group in Example 1 (animal number G1-317, 100x magnification).
Figure 10 shows the staining results of the skin pathological section of the model group in Example 1 (animal number G2-315, 100x magnification).
Figure 11 shows the staining results of the skin pathological section of the model group in Example 1 (animal number G2-331, 100x magnification).
Figure 12 shows the staining results of the skin pathological section of the model group in Example 1 (animal number G2-382, 100x magnification).
Figure 13 shows the staining results of pathological sections of the skin in the dexamethasone treatment group in Example 1 (animal number G3-312, 100x magnification).
Figure 14 shows the staining results of pathological sections of the skin in the dexamethasone treatment group in Example 1 (animal number G3-333, 100x magnification).
Figure 15 shows the staining results of pathological sections of the skin in the dexamethasone treatment group in Example 1 (animal number G3-334, 100x magnification).
Figure 16 shows the staining results of the skin pathological section of the Nib1 treatment group in Example 1 (animal number G4-322, 100x magnification).
Figure 17 shows the staining results of the skin pathological section of the Nib1 treatment group in Example 1 (animal number G4-328, 100x magnification).
Figure 18 shows the staining results of the skin pathological section of the Nib1 treatment group in Example 1 (animal number G3-346, 100x magnification).
Figure 19 shows the scoring results of the pathological section in Example 1.
Fig. 20 shows the change of animal body weight in the disease model of Example 2.
Figure 21 shows the rash score of the disease model in Example 2.
Figure 22 shows the peeling symptom score of the disease model in Example 2.
Figure 23 shows the skin thickening symptom score of the disease model in Example 2.
Figure 24 is the total clinical score of the disease model in Example 2.
Figure 25 shows the results of the area under the total clinical score curve (AUC) in Example 2.
Figure 26 is a pathological section of the skin of the normal group in Example 2 (animal G1-3, 100x magnification, H&E staining).
Figure 27 is a pathological section of the skin of the normal group in Example 2 (animal G1-25, 100x magnification, H&E staining).
Figure 28 is a skin pathological section of the normal group in Example 2 (animal G1-57, 100x magnification, H&E staining).
Figure 29 is a pathological section of the skin of the treatment group of the external application preparation in Example 2 (animal G5-5, 100x magnification, H&E staining).
Figure 30 is a pathological section of the skin of the treatment group of the external application preparation in Example 2 (animal G5-43, 100x magnification, H&E staining).
Figure 31 is a pathological section of the skin of the treatment group of the external application preparation in Example 2 (animal G5-9, 100x magnification, H&E staining).
Figure 32 is a skin pathological section of the control group of the external application preparation of Example 2 (animal G6-10, 100x magnification, H&E staining).
Figure 33 is a skin pathological section of the control group of the external application preparation of Example 2 (animal G6-21, 100x magnification, H&E staining).
Figure 34 is a skin pathological section of the control group of the external application preparation of Example 2 (animal G6-28, 100x magnification, H&E staining).

### Detailed Description of the Embodiments

The present invention will be described further in reference to the examples.

Efficacy verification of Nib1 in animal models of psoriasis

### Example 1. Treatment of psoriasis by oral administration of Nib1

### 1. Reagents

5% Imiquimod ointment, Aldara, 3M Pharmaceuticals; Dexamethasone ointment: Shanghai Sanjiu Pharmaceutical; PEG300, Sigma, catalog number 90878. Nib1(pimozide): Sigma (P1793).

### 2. Protocol

Female BALB/c mice (purchased from Shanghai SLACK Laboratory Animal Co., Ltd, administered at 6 weeks of age) were randomly divided into groups after adapting to the environment. Except for the normal control group, the rest of the animals received imiquimod ointment (5%) externally to induce the establishment of a psoriasis model. Specifically, the mice were shaved off same area of the back hair and applied with 62.5 mg of imiquimod ointment daily for seven consecutive days. Animals received different treatment programs, weighed daily, orally or externally applied with drugs once, and recorded with disease progression score.

The specific grouping information is shown in Table 1. Both the vehicle control group and the Nib1 treatment group received oral gavage of the same volume of drugs, formulated with 30% PEG300, and the Nib1 group was given a dose of 10 mg/kg. Dexamethasone ointment was used as a positive treatment drug and received 70 mg smear treatment once a day. The disease progression score is evaluated on a scale of 0-4 based on three indicators, rash, peeling, and thickening (0: no symptoms; 1: mild symptoms; 2: moderate symptoms; 3: significant symptoms; 4: very significant symptoms). At the end of the experiment, the animals were sacrificed, and pathological section analysis was performed on the skin samples of the back lesions. The scoring standards are shown in Table 2.

**Table 1. Groups and Treatments**

| Groups | Animals (n) | Group Names | Modeling agent for external use | Treatment dose | Dosing Regimen | |
|---|---|---|---|---|---|---|
| | | | | | Routes | Frequency |
| G1 | 5 | Normal | None | None | None | None |
| G2 | 10 | Vehicle PO | 62.5 mg 5% imiquimod ointment, once a day for seven consecutive days | None | oral | Once a day for 7 consecutive days |
| G3 | 10 | DEX cream Topical | | 70mg | external | Once a day for 7 consecutive days |
| G4 | 10 | Nib1 10mpk PO | | 10mg/kg | oral | Once a day for 7 consecutive days |

**Table 2. Scoring Standards of pathological sections**

| | | |
|---|---|---|
| | Items | Scores |
| Corneum | abscess | 1.5 |
| | parakeratosis | 0.5-1.5 |
| | hyperkeratosis | 0.5 |
| Epidermis | length of omentum ridge | 0.5-1.5 |
| | acanthosis | 1 |
| | lack of stratum granulosum | 1 |
| Dermis | immune cell filtration in dermis | 0.5-1 |
| | papilla blockage | 1 |
| | papilla thinning | 0.5 |

### 3. Results

### 3.1 Body weights

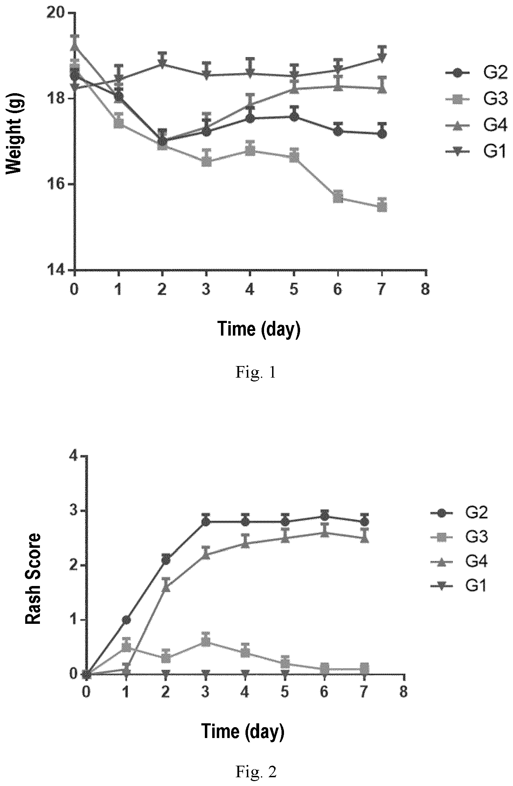

As shown in Figure 1 and Table 3, the body weights of the animals in the dexamethasone topical treatment group were significantly reduced, indicating that the drug has greater toxic and side effects, while in the Nib1 oral group, body weights decreased slightly in the first two days, but recovered quickly, which was similar to the control group and within the acceptable range, suggesting Nib1 has a toxic and side effect significantly less than that of dexamethasone.

**Table 3. Statistical significance analysis of body weight**

| Group/day | 0 | 1 | 2 | 3 | 4 | 5 | 6 | 7 |
|---|---|---|---|---|---|---|---|---|
| DEX vs. Vehicle | ns | ^{∗} | ns | ns | ^{∗} | ^{∗∗} | ^{∗∗∗∗} | ^{∗∗∗∗} |
| Nib1 vs Vehicle | * | ns | ns | ns | ns | * | ** | ** |

| | | | | | | | | |
|---|---|---|---|---|---|---|---|---|
| ^{∗}p<0.05, ^{∗∗}p<0.01, ^{∗∗∗}p<0.001, ^{∗∗∗∗}p<0.0001, t-test, compared with the vehicle control group, ns means no statistically significant difference. | | | | | | | | |

### 3.2. Nib1 oral treatment improves symptoms of the disease

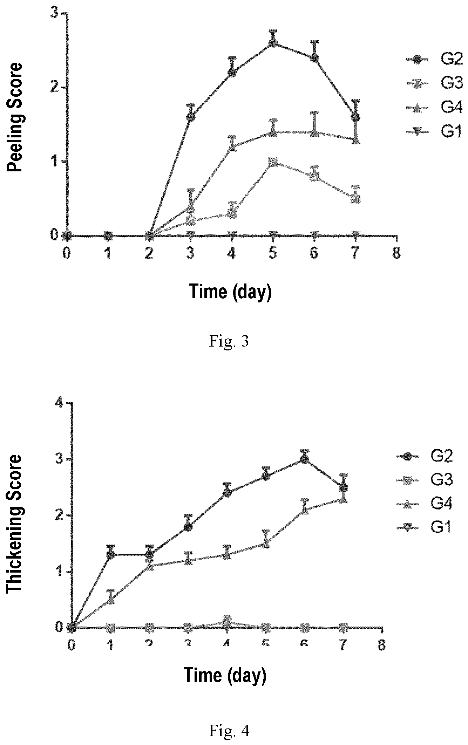

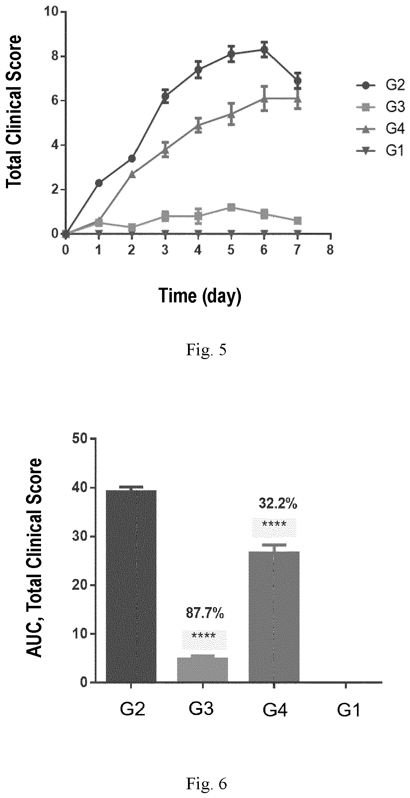

The clinical scoring of three indicators including skin rash, peeling and thickening were recorded and shown as statistical average value and ± standard error by day. The results are shown in Figures 2-4 and Tables 4-6. With daily use of imiquimod ointment on the skin, the model group showed gradually increasing clinical symptoms, and the rash reached a peak around the 3rd day and remained until the end of the model. The Nib1 oral administration group showed a slightly reduced rash, which was significantly different in the first three days (Figure 2, Table 4). The peeling phenomenon in the model group gradually increased with time, reached a peak on the fifth day, and then decreased. Nib1 group could significantly reduce the peeling phenomenon, reaching a peak on the 4th day and maintaining a relatively low level to the end point (Figure 3, Table 5). The skin thickening of the model group had been increasing, while the Nib1 group showed the same trend, but the thickening level was significantly reduced on the 4th, 5th, and 6th days (Figure 4, Table 6). The total score of the three indicators showed that the Nib 1 group had significantly lower clinical symptoms than the disease model group (Figure 5, Table 7).

**Table 4. statistical significance analysis of rash scores**

| Group/day | 0 | 1 | 2 | 3 | 4 | 5 | 6 | 7 |
|---|---|---|---|---|---|---|---|---|
| DEX vs. Vehicle | ns | ** | ^{∗∗∗∗} | ^{∗∗∗∗} | ^{∗∗∗∗} | ^{∗∗∗∗} | ^{∗∗∗∗} | ^{∗∗∗∗} |
| Nib1 vs Vehicle | ns | **** | * | ** | ns | ns | ns | ns |

| | | | | | | | | |
|---|---|---|---|---|---|---|---|---|
| ^{∗}p<0.05, ^{∗∗}p<0.01, ^{∗∗∗}p<0.001, ^{∗∗∗∗}p<0.0001, t-test, compared with the vehicle control group, ns means no statistically significant difference. | | | | | | | | |

**Table 5. statistical significance analysis of peeling scores**

| Group/day | 0 | 1 | 2 | 3 | 4 | 5 | 6 | 7 |
|---|---|---|---|---|---|---|---|---|
| DEX vs. Vehicle | ns | ns | ns | ^{∗∗∗∗} | ^{∗∗∗∗} | ^{∗∗∗∗} | ^{∗∗∗∗} | *** |
| Nib1 vs Vehicle | ns | ns | ns | *** | *** | **** | ** | ns |

| | | | | | | | | |
|---|---|---|---|---|---|---|---|---|
| ^{∗}p<0.05, ^{∗∗}p<0.01, ^{∗∗∗}p<0.001, ^{∗∗∗∗}p<0.0001, t-test, compared with the vehicle control group, ns means no statistically significant difference. | | | | | | | | |

**Table 6. statistical significance analysis of skin thickening scores**

| | | | | | | | | |
|---|---|---|---|---|---|---|---|---|
| Group/day | 0 | 1 | 2 | 3 | 4 | 5 | 6 | 7 |
| DEX vs. | ns | ^{∗∗∗∗} | ^{∗∗∗∗} | ^{∗∗∗∗} | ^{∗∗∗∗} | ^{∗∗∗∗} | ^{∗∗∗∗} | ^{∗∗∗∗} |
| Vehicle | | | | | | | | |
| Nib1 vs Vehicle | ns | ^{∗∗} | ns | ^{∗} | ^{∗∗∗} | ^{∗∗∗} | ^{∗∗} | ns |

| | | | | | | | | |
|---|---|---|---|---|---|---|---|---|
| ^{∗}p<0.05, ^{∗∗}p<0.01, ^{∗∗∗}p<0.001, ^{∗∗∗∗}p<0.0001, t-test, compared with the vehicle control group, ns means no statistically significant difference. | | | | | | | | |

The results of comprehensive evaluation of the three clinical indicators are shown in Figure 5 and Table 7. Nib1 oral treatment can significantly reduce the clinical symptoms of psoriasis.

**Table 7. statistical significance analysis of total clinical scores**

| Group/day | 0 | 1 | 2 | 3 | 4 | 5 | 6 | 7 |
|---|---|---|---|---|---|---|---|---|
| DEX vs. Vehicle | ns | ^{∗∗∗∗} | ^{∗∗∗∗} | ^{∗∗∗∗} | ^{∗∗∗∗} | ^{∗∗∗∗} | ^{∗∗∗∗} | ^{∗∗∗∗} |
| Nib1 vs Vehicle | ns | **** | ** | **** | **** | *** | ** | ns |

| | | | | | | | | |
|---|---|---|---|---|---|---|---|---|
| ^{∗}p<0.05, ^{∗∗}p<0.01, ^{∗∗∗}p<0.001, ^{∗∗∗∗}p<0.0001, t-test, compared with the vehicle control group, ns means no statistically significant difference. | | | | | | | | |

The calculation result of the area under the clinical total score curve (AUC) is shown in Figure 6, showing the quantitative reduction effect of the curative effect. Compared with the disease model group, the oral administration of Nib1 could significantly reduce the disease progression, and the quantitative analysis indicated a 32.2% remission.

### 3.3 Results of pathological sections

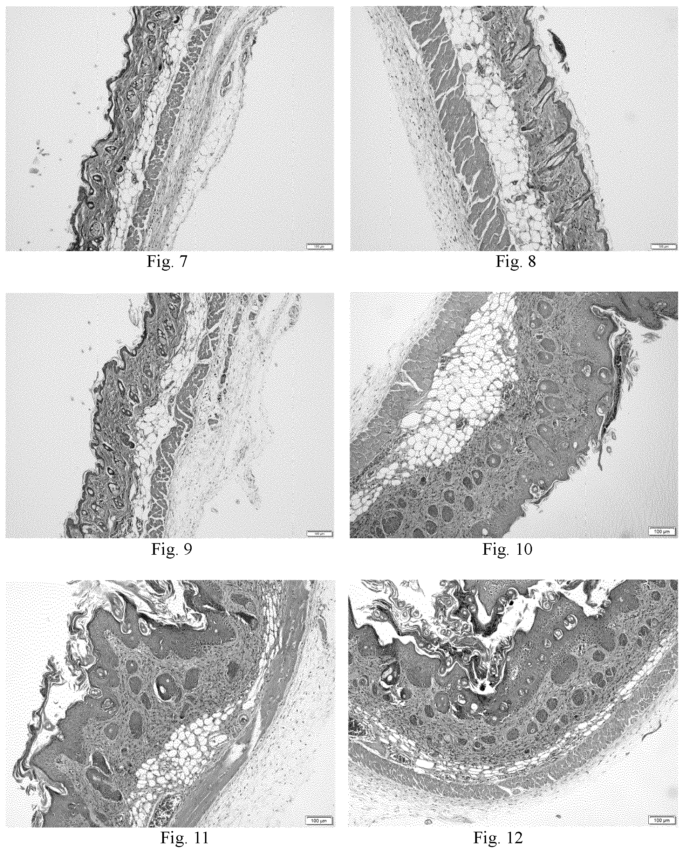

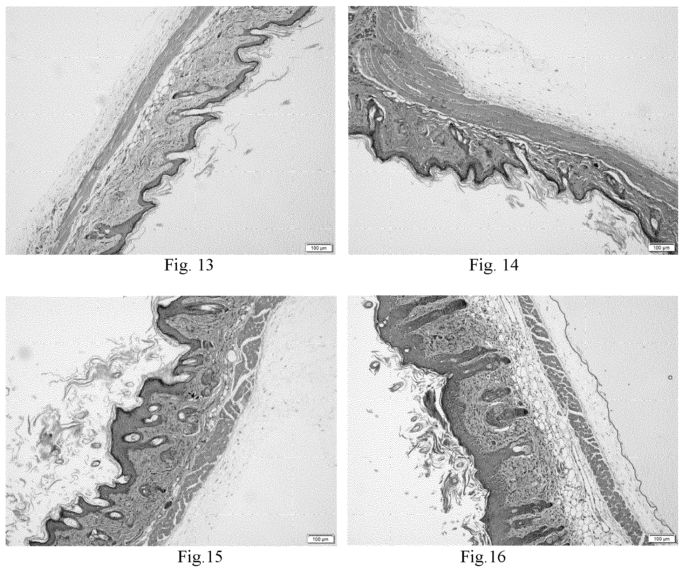

The pathological sections of animal skin were stained with eosin. The 100x magnified pictures are shown in Figures 7-18. Three samples were selected for each group. Compared with the normal control group, the disease model group showed significant epidermal hyperplasia, immune cell infiltration, destruction of the epidermal layered structure, and abnormal blood vessel structure. Nib1 group can improve epidermal hyperplasia and reduce immune cell infiltration.

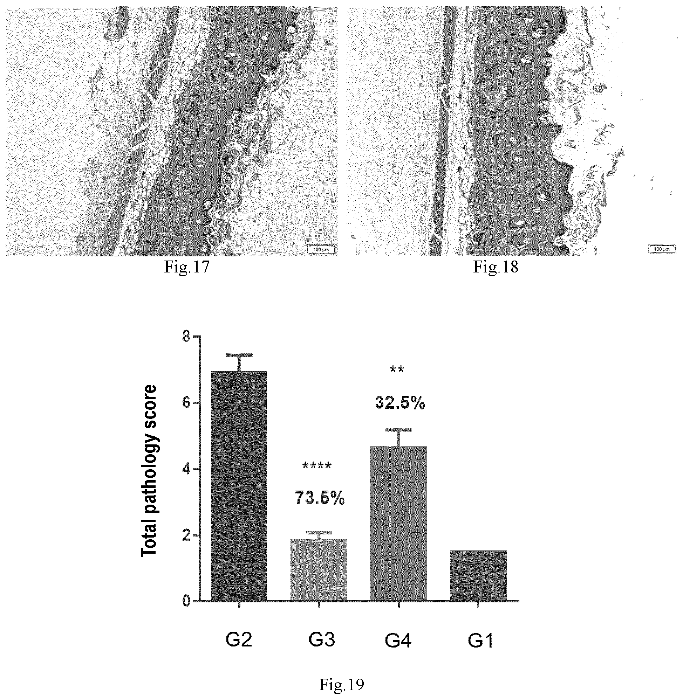

The pathological sections were scored according to the scoring system in Table 2, and the results are shown in Figure 19. Nib1 treatment group showed a statistically significant treatment effect, reaching a 32.5% remission effect (p<0.01).

Note: The data were shown as average value ± standard error. ^{∗}p<0.05, ^{∗∗}p<0.01, ^{∗∗∗}p<0.001, ^{∗∗∗∗}p<0.0001, one-way ANOVA, compared with the vehicle control group.

### Example 2. Therapeutic effect of Nib1 for external use on animal psoriasis disease model

### 1. Reagents

5% Imiquimod ointment, Aldara, 3M Pharmaceuticals, H20160079; Dexamethasone ointment: Shanghai Sanjiu Pharmaceutical. Cetylstearyl alcohol, purchased from Hunan Erkang Pharmaceutical, F20170000297.

### 2. Preparation for external application

There are two formulations designed for the preparation for external application, and the formulation FLL-15-21-2 is shown in Table 8.

**Table 8. Composition of formulation FLL-15-21-2**

| Formulation FLL-15-21-2 | | | | |
|---|---|---|---|---|
| Materials | Effects | Content (mg) | Proportions % | Batch (g) |
| Nib1 | API | 25.0 | 0.5% | 0.25 |
| acetic acid | solvents and salt-forming excipients of API | 40.0 | 0.8% | 0.40 |
| cetostearyl alcohol | oil phase matrix for solidification | 550.0 | 11.0% | 5.50 |
| medium chain triglycerides | ordinary oil phase solution | 450.0 | 9.0% | 4.50 |
| Tween 60 | emulsifier | 150.0 | 3.0% | 1.50 |
| Span 80 | emulsifier | 35.0 | 0.7% | 0.35 |
| dimethyl sulfoxide | solvents and penetration enhancers for API | 300.0 | 6.0% | 3.00 |
| benzyl alcohol | solvents and preservatives for API | 450.0 | 9.0% | 4.50 |
| purified water | water | 3000.0 | 60.0% | 30.00 |
| Total | / | 5000.0 | 100.0% | 50.00 |

The formulation FLL-15-21-2 of the preparation for external application is formulated as follows.
(1) weighing the prescription amount of API in a beaker, and then adding the prescription amount of acetic acid; when adding, the acetic acid just surpasses the API powder, and the whole is changed from a white solid to a slightly sticky crystal; then adding a mixed solvent of the prescription amounts of dimethyl sulfoxide and benzyl alcohol, a small part of the transparent crystal being dissolved by stirring at room temperature, and then the whole being placed in a water bath at 70°C for about 5 minutes; the API was dissolved into the solvent; after cooling, the API did not precipitate.
(2) adding the prescription amount of medium-chain triglycerides to the above solution, the API did not precipitate; then adding the prescription amount of cetostearyl alcohol and the rest of emulsifiers, and placing it in a 70°C water bath for about 15 minutes to obtain a transparent oil phase solution; at the same time, putting the water in a 70°C water bath to keep warm.
(3) adding water at the same temperature to the above oil phase solution; the mixture became a white emulsion after the addition; taking it out and keeping stirring until it cooled to become a cream; finally, a pH test paper was used to to roughly detect the pH of the preparation of 5-6.

The control formulation FLL-15-53-2 is the corresponding formulation lack of the API.

Formulation FLL-15-25-2 is shown in Table 9.

**Table 9. Composition of formulation FLL-15-25-2**

| Formulation FLL-15-25-2 | | | | |
|---|---|---|---|---|
| Materials | Effects | Content (mg) | Proportions % | Batch (g) |
| Nib1 | API | 25.0 | 0.5% | 0.25 |
| cetostearyl alcohol | solidified oil phase | 550.0 | 11.0% | 5.50 |
| medium chain triglycerides | ordinary oil phase | 450.0 | 9.0% | 4.50 |
| Tween 60 | emulsifier | 150.0 | 3.0% | 1.50 |
| Span 80 | emulsifier | 50.0 | 1.0% | 0.50 |
| dimethyl sulfoxide | solvents and penetration enhancers for API | 300.0 | 6.0% | 3.00 |
| benzyl alcohol | solvents and preservatives for API | 450.0 | 9.0% | 4.50 |
| purified water | water phase | 3025.0 | 60.5% | 30.25 |
| Total, g | / | 5000.0 | 100.0% | 50.00 |

The formulation FLL-15-25-2 of the preparation for external application is formulated as follows.
(1) weighing the prescribed amount of API and adding it to the mixed solvent composed of dimethyl sulfoxide and benzyl alcohol, shaking and stirring for a while, and placing it in a water bath at 70°C for about 19 minutes to obtain an oil phase solution of the API;
(2) adding oil phases including medium-chain triglycerides, cetostearyl alcohol, Tween 60, and Span 80 to the above API-containing solution, and then heating and melting in a 70°C water bath to obtain a transparent API-containing solution; no API was precipitated; and at the same time, heating the water at 70°C;
(3) adding the above water at the same temperature to the transparent oil phase solution; the whole system became a milky white emulsion at the moment of the adding; keeping stirring, and after taking it out, stirring to cool, and finally it became a milky white cream; API crystals were precipitated as observed under microscope.

The control formulation FLL-15-55-2 is the corresponding formulation lack of the API.

### 3. Protocols

After adapting to the environment, female BALB/c mice were randomly divided into groups. Except for the normal control group, the rest received external treatment with imiquimod ointment (5%) to induce the establishment of a psoriasis model. Specifically, the mice were shaved off the same area (2×3 cm) of back hair and applied with 62.5 mg ointment daily for seven consecutive days. The animals received different treatments, weighed daily, received 70mg smear treatment once, and recorded with disease progression score. Grouping information is shown in Table 10. The disease progression score is evaluated on a scale of 0-4 based on three indicators, rash, peeling, and thickening (0: no symptoms; 1: mild symptoms; 2: moderate symptoms; 3: significant symptoms; 4: very significant symptoms). At the end of the experiment, the animals were sacrificed, and pathological section analysis was performed on the skin samples of the back lesions.

**Table 10. Groups and Treatments**

| Groups | Animals (n) | Group Names | Modeling agent for external use | Treatment dose | Dosing Regimen | |
|---|---|---|---|---|---|---|
| | | | | | Routes | Frequencies |
| 1 | 3 | Normal | None | None | None | None |
| 2 | 8 | Dexamethasone topical treatment group (DEX) | 62.5 mg 5% imiquimod ointment, once a day for seven consecutive days | 70mg | external | once a day for 7 consecutive days |
| 3 | 8 | FLL-15-21-2 treatment group | | 70mg | external | once a day for 7 consecutive days |
| 4 | 8 | FLL-15-53-2 control group | | 70mg | external | once a day for 7 consecutive days |
| 5 | 8 | FLL-15-25-2 treatment group | | 70mg | external | once a day for 7 consecutive days |
| 6 | 8 | FLL-15-55-2 control group | | 70mg | external | once a day for 7 consecutive days |

### 4. Results

### 4.1 Body weight changes

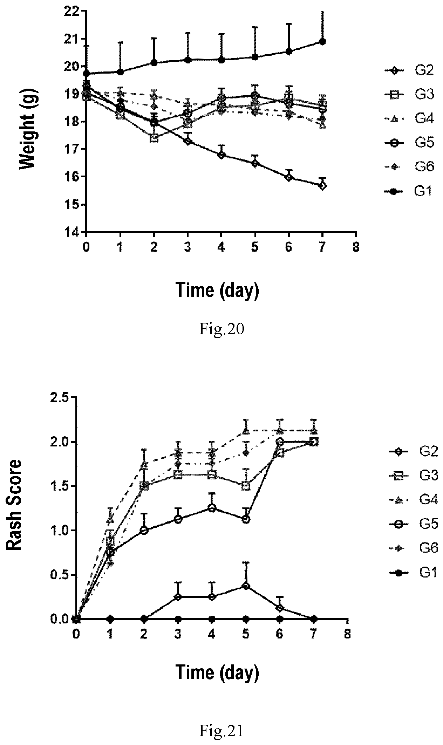

As shown in Figure 20, the weight loss of the dexamethasone ointment treatment group was more than 25%. Compared with the external preparation control group, the animal body weight of each Nib1 external preparation group did not show significant changes, indicating that the side effects of the two preparations were not obvious, and their safety was far better than that of dexamethasone ointment.

### 4.2 Evaluation of the clinical symptoms of the disease

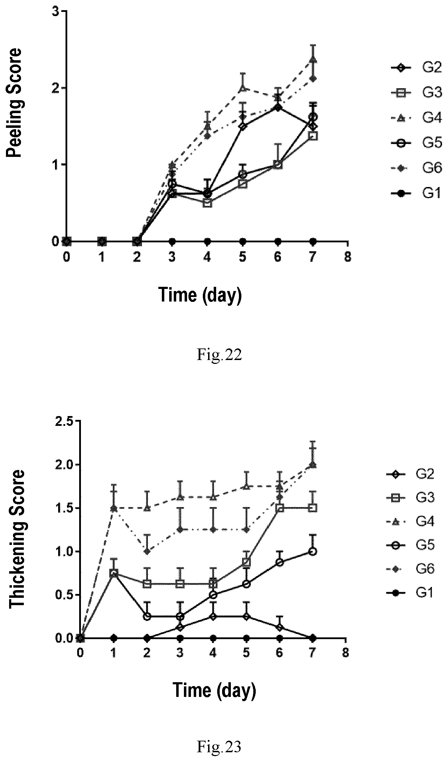

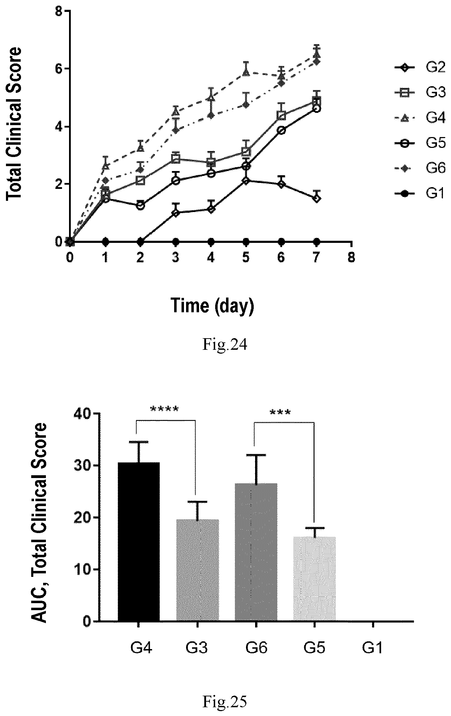

As shown in Figures 21-25, the psoriasis model was successfully established in mice, showing symptoms of severe red rash, peeling and thickening of the skin. Nib1 external application preparation FLL-15-25-2 performed better than FLL-15-21-2 in reducing rash and skin thickening symptoms, and both were better than the dexamethasone ointment treatment group in improving the symptoms of peeling. Overall, the two kinds of external applications have significant curative effect than the corresponding control preparation. ^{∗∗∗∗}p<0.0001, ^{∗∗∗}p<0.001.

### 4.3 Pathological section results

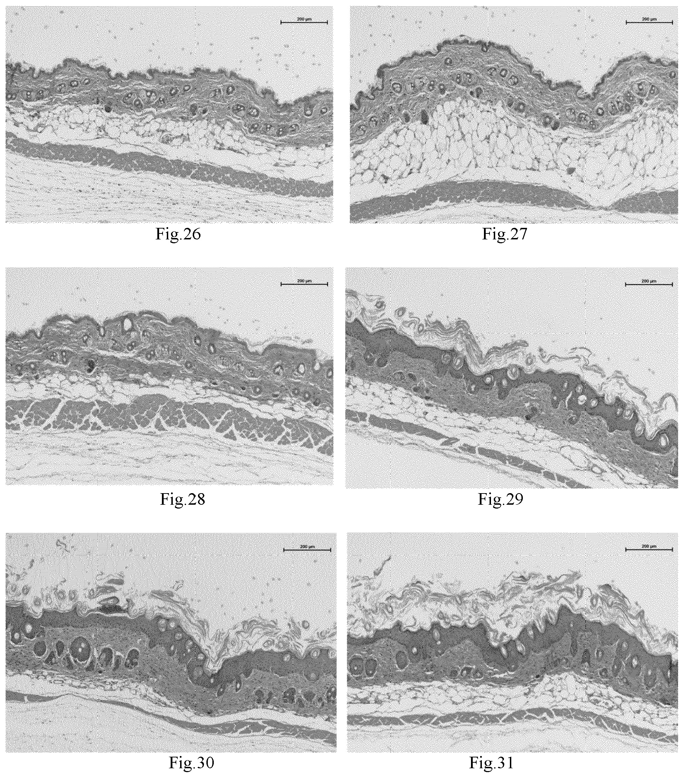

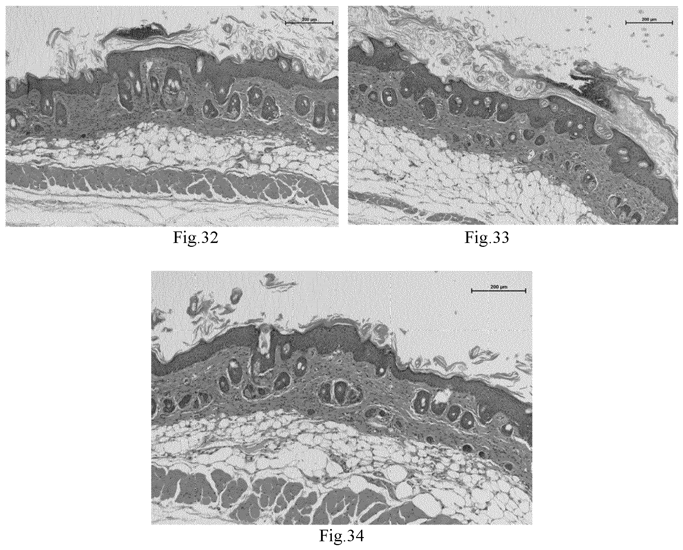

The pathological sections of animal skin were stained, and the 100-fold magnified pictures are shown in Figures 26-34. Three samples were selected for each group. Compared with the healthy control group, the external application control group showed pathological changes such as thickening of the epidermis, vasodilatation, and immune cell infiltration, while the external treatment group showed significant improvement in the above symptoms.

The raw materials and equipment used in the present invention, unless otherwise specified, are all commonly used raw materials and equipment in the field; the methods used in the present invention, unless otherwise specified, are all conventional methods in the field.

The above are only preferred embodiments of the present invention and do not have any limitation on the present invention. Any simple modifications, changes and equivalent made to the above embodiments according to the technical spirit of the present invention still are within the scope of invention.

## Claims

1. A compound Nib1, a pharmaceutically acceptable salt thereof, a solvate thereof, a solvate of a pharmaceutically acceptable salt thereof, or a crystal form thereof, for use in one or more of the following:
(a) treatment of an inflammation induced autoimmune skin disease;
(b) improvement of skin rash;
(c) improvement of skin peeling;
(d) reduction of skin thickening;
(e) reduction of epidermal hyperplasia;
(f) improvement of blood vessel dilation; and
(g) reduction of immune cell infiltration;
wherein the compound Nib 1 has a structure of 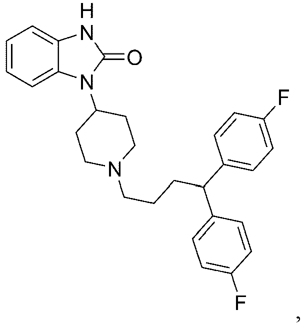 and
preferably, the inflammation induced autoimmune skin disease is non-specific dermatitis, such as psoriasis, scleroderma, hydroa, and/or alopecia areata.

2. Use of a compound Nib1, a pharmaceutically acceptable salt thereof, a solvate thereof, a solvate of a pharmaceutically acceptable salt thereof, or a crystal form thereof, in the preparation of a medicament for treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration;
wherein the compound Nib 1 has a structure of 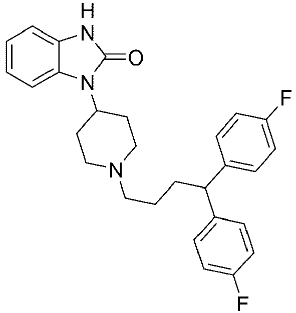 , and
preferably, the inflammation induced autoimmune skin disease is non-specific dermatitis, such as psoriasis, scleroderma, hydroa, and/or alopecia areata.

3. A pharmaceutical composition, comprising a compound Nib1, a pharmaceutically acceptable salt thereof, a solvate thereof, a solvate of a pharmaceutically acceptable salt thereof, or a crystal form thereof, for use in one or more of the following:
(a) treatment of an inflammation induced autoimmune skin disease;
(b) improvement of skin rash;
(c) improvement of skin peeling;
(d) reduction of skin thickening;
(e) reduction of epidermal hyperplasia;
(f) improvement of blood vessel dilation; and
(g) reduction of immune cell infiltration;
wherein the compound Nib 1 has a structure of 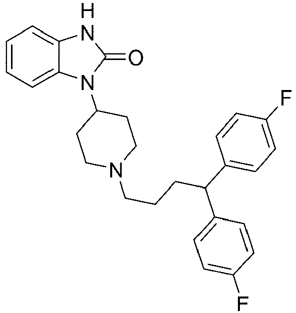 , and
preferably, the inflammation induced autoimmune skin disease is non-specific dermatitis, such as psoriasis, scleroderma, hydroa, and/or alopecia areata;
and/or, the pharmaceutical composition further comprises a pharmaceutically acceptable carrier, excipient and/or solvent;
and/or, the pharmaceutical composition further comprises a further medicament for treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration.

4. The pharmaceutical composition of claim 3, wherein the pharmaceutical composition is in the form of a preparation for internal or external use;
and/or, the pharmaceutical composition is in the form of an oral preparation or a preparation for external application; the compound Nib1, a pharmaceutically acceptable salt thereof, a solvate thereof, a solvate of a pharmaceutically acceptable salt thereof, or a crystal form thereof, in the oral preparation, is administered at preferably 0.1-1.098 mg/kg per time, once a day; the compound Nib1, a pharmaceutically acceptable salt thereof, a solvate thereof, a solvate of a pharmaceutically acceptable salt thereof, or a crystal form thereof, in the preparation for external application is administered at 5-15 mg/cm² each time, at least once a day.

5. The pharmaceutical composition of claim 4, wherein when the pharmaceutical composition is in the form of a preparation for external application, the preparation for external application comprises, by weight, 0.2-0.3 parts of the compound Nib1, a pharmaceutically acceptable salt thereof, a solvate thereof, a solvate of a pharmaceutically acceptable salt thereof, or a crystal form thereof, 9-11 parts of an oil phase, 1.3-2.6 parts of an emulsifier, 6.5-8.5 parts of a solvent and 25-35 parts of water;
preferably,
the compound Nib1, a pharmaceutically acceptable salt thereof, a solvate thereof, a solvate of a pharmaceutically acceptable salt thereof, or a crystal form thereof is 0.25 parts by weight;
and/or, the oil phase includes one or more oil phases, preferably two oil phases;
and/or, the oil phase includes a solidified oil phase and an ordinary oil phase; the solidified oil phase is preferably 5-6 parts, such as 5.5 parts, and the ordinary oil phase is preferably 4-5 parts, such as 4.5 parts; the solidified oil phase is preferably cetostearyl alcohol, and the ordinary oil phase is preferably medium chain triglycerides;
and/or, the emulsifier includes one or more emulsifiers, and preferably includes Tween 60 and Span 80, wherein the weight of Tween 60 is preferably 1-2 parts, such as 1.5 parts, and the weight of Span 80 is preferably 0.3-0.6 parts, for example 0.35, 0.4 or 0.5 parts;
and/or, the solvent includes one or more solvents, preferably including dimethyl sulfoxide and benzyl alcohol, wherein the weight of the dimethyl sulfoxide is preferably 2.5-3.5 parts, such as 3 parts, and the weight of benzyl alcohol is preferably 4-5 parts, for example 4.5 parts;
and/or, the weight of the water is preferably 30 parts or 30.25 parts;
and/or, the preparation for external application further includes acetic acid, and the weight of the acetic acid is preferably 0.3-0.5 parts, more preferably 0.4 parts;
more preferably,
the preparation for external application comprises by weight 0.25 parts of the compound Nib1, a pharmaceutically acceptable salt thereof, a solvate thereof, a solvate of a pharmaceutically acceptable salt thereof, or a crystal form thereof, 0.4 parts of acetic acid, 5.5 parts of cetostearyl alcohol, 4.5 parts of medium chain triglycerides, 1.5 parts of Tween 60, 0.35 parts of Span 80, 3 parts of dimethyl sulfoxide, 4.5 parts of benzyl alcohol, and 30 parts of water; alternatively, the preparation for external application comprises by weight 0.25 parts of the compound Nib1, a pharmaceutically acceptable salt thereof, a solvate thereof, a solvate of a pharmaceutically acceptable salt thereof, or a crystal form thereof, 5.5 parts of cetostearyl alcohol, 4.5 parts of medium chain triglycerides, 1.5 parts of Tween 60, 0.5 parts of Span 80, 3 parts of dimethyl sulfoxide, 4.5 parts of benzyl alcohol, and 30.25 parts of water.

6. The pharmaceutical composition of claim 5, wherein the preparation for external application is prepared by mixing the compound Nib 1, a pharmaceutically acceptable salt thereof, a solvate thereof, a solvate of a pharmaceutically acceptable salt thereof, or a crystal form thereof, the oil phase, the emulsifier, the solvent, and water;
preferably, the preparation for external application can be prepared by first dissolving the compound Nib1, a pharmaceutically acceptable salt thereof, a solvate thereof, a solvate of a pharmaceutically acceptable salt thereof, or a crystal form thereof, in the solvent, followed by adding the oil phase and the emulsifier, and finally adding water;
more preferably,
the temperature of the dissolution is 65-75°C; and/or, the dissolution time is 4-6 min; and/or, after adding the oil phase and the emulsifier, it is placed in an environment with a temperature of preferably 65-75°C for preferably 10-20 min; and/or, the temperature of the water is 65-75°C; and/or, after adding the water, the mixture is stirred, preferably until it is cooled to form a cream.

7. Use of compound Nib1, a pharmaceutically acceptable salt thereof, a solvate thereof, a solvate of a pharmaceutically acceptable salt thereof, or a crystal form thereof, of claim 1 or 2, or the pharmaceutical composition of any one of claims 3 to 6, for the treatment of an inflammation induced autoimmune skin disease; or for improvement of skin rash, improvement of skin peeling, reduction of skin thickening, reduction of epidermal hyperplasia, improvement of blood vessel dilation and/or reduction of immune cell infiltration;
preferably, the inflammation induced autoimmune skin disease is non-specific dermatitis, such as psoriasis, scleroderma, hydroa, and/or alopecia areata.

8. Use of compound Nib1, a pharmaceutically acceptable salt thereof, a solvate thereof, a solvate of a pharmaceutically acceptable salt thereof, or a crystal form thereof, of claim 1 or 2, or the pharmaceutical composition of any one of claims 3 to 6, in the preparation of a medicament for the treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration;
preferably,
the inflammation induced autoimmune skin disease is non-specific dermatitis, such as psoriasis, scleroderma, hydroa, and/or alopecia areata; and/or, the medicament further comprises a pharmaceutically acceptable carrier, an excipient and/or a solvent; and/or, the medicament is in the form of a pharmaceutical composition which preferably further comprises a further drug for treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration.

9. A method for treatment of an inflammation induced autoimmune skin disease, improvement of skin rash, improvement of skin peeling, reduction of skin thickening, reduction of epidermal hyperplasia, improvement of blood vessel dilation and/or reduction of immune cell infiltration, comprising using the compound Nib1, a pharmaceutically acceptable salt thereof, a solvate thereof, a solvate of a pharmaceutically acceptable salt thereof, or a crystal form thereof, of claim 1 or 2, or the pharmaceutical composition of any one of claims 3 to 6, for the treatment;
preferably, the inflammation induced autoimmune skin disease is non-specific dermatitis, such as psoriasis, scleroderma, hydroa, and/or alopecia areata.

10. A preparation for external application, comprising, by weight, 0.2-0.3 parts of compound Nib1, a pharmaceutically acceptable salt thereof, a solvate thereof, a solvate of a pharmaceutically acceptable salt thereof, or a crystal form thereof, 9-11 parts of oil phase, 1.3-2.6 parts of emulsifier, 6.5-8.5 parts of solvent, and 25-35 parts of water;
preferably,
the preparation for external application is useful for one or more of the following: (a) treatment of an inflammation induced autoimmune skin disease; (b) improvement of skin rash; (c) improvement of skin peeling; (d) reduction of skin thickening; (e) reduction of epidermal hyperplasia; (f) improvement of blood vessel dilation; and (g) reduction of immune cell infiltration;
and/or, the compound Nib1, a pharmaceutically acceptable salt thereof, a solvate thereof, a solvate of a pharmaceutically acceptable salt thereof, or a crystal form thereof is 0.25 parts by weight;
and/or, the oil phase includes one or more oil phases, preferably two oil phases;
and/or, the oil phase includes a solidified oil phase and an ordinary oil phase. The solidified oil phase is preferably 5-6 parts, such as 5.5 parts, and the ordinary oil phase is preferably 4-5 parts, such as 4.5 parts. The solidified oil phase is preferably cetostearyl alcohol, and the ordinary oil phase is preferably medium chain triglycerides;
and/or, the emulsifier includes one or more emulsifiers, and preferably includes Tween 60 and Span 80, wherein the weight of Tween 60 is preferably 1-2 parts, such as 1.5 parts, and the weight of Span 80 is preferably 0.3-0.6 parts, for example 0.35, 0.4 or 0.5 parts;
and/or, the solvent includes one or more solvents, preferably including dimethyl sulfoxide and benzyl alcohol, wherein the weight of the dimethyl sulfoxide is preferably 2.5-3.5 parts, such as 3 parts, and the weight of benzyl alcohol is preferably 4-5 parts, for example 4.5 parts;
and/or, the weight of the water is preferably 30 parts or 30.25 parts;
and/or, the preparation for external application further includes acetic acid, and the weight of the acetic acid is preferably 0.3-0.5 parts, more preferably 0.4 parts;
more preferably,
the preparation for external application comprises by weight 0.25 parts of the compound Nib1, a pharmaceutically acceptable salt thereof, a solvate thereof, a solvate of a pharmaceutically acceptable salt thereof, or a crystal form thereof, 0.4 parts of acetic acid, 5.5 parts of cetostearyl alcohol, 4.5 parts of medium chain triglycerides, 1.5 parts of Tween 60, 0.35 parts of Span 80, 3 parts of dimethyl sulfoxide, 4.5 parts of benzyl alcohol, and 30 parts of water; alternatively, the preparation for external application comprises by weight 0.25 parts of the compound Nib1, a pharmaceutically acceptable salt thereof, a solvate thereof, a solvate of a pharmaceutically acceptable salt thereof, or a crystal form thereof, 5.5 parts of cetostearyl alcohol, 4.5 parts of medium chain triglycerides, 1.5 parts of Tween 60, 0.5 parts of Span 80, 3 parts of dimethyl sulfoxide, 4.5 parts of benzyl alcohol, and 30.25 parts of water.

11. The preparation for external application of claim 10, wherein the preparation for external application is prepared by mixing the compound Nib1, a pharmaceutically acceptable salt thereof, a solvate thereof, a solvate of a pharmaceutically acceptable salt thereof, or a crystal form thereof, the oil phase, the emulsifier, the solvent, and water;
preferably,
the preparation for external application is prepared by first dissolving the compound Nib1, a pharmaceutically acceptable salt thereof, a solvate thereof, a solvate of a pharmaceutically acceptable salt thereof, or a crystal form thereof, in the solvent, followed by adding the oil phase and the emulsifier, and finally adding water; more preferably, the temperature of the dissolution is 65-75°C; and/or, the dissolution time is 4-6 min; and/or, after adding the oil phase and the emulsifier, it is placed in an environment with a temperature of preferably 65-75°C for preferably 10-20 min; and/or, the temperature of the water is 65-75°C; and/or, after adding the water, the mixture is stirred, preferably until it is cooled to form a cream.

12. A medicament with low side effects for treatment of psoriasis, comprising compound Nib1 and/or a pharmaceutically acceptable salt thereof, wherein compound Nib1 has chemical formula of C₂₈H₂₉F₂N₃O and a structure of

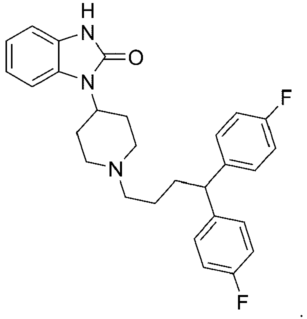

13. The medicament for treatment of psoriasis of claim 12, wherein the medicament further comprises a pharmaceutically acceptable carrier, an excipient and/or a solvent;
preferably, the medicament is in the form of a preparation for internal or external use.

14. The medicament for treatment of psoriasis of claim 13, wherein the medicament is in the form of an oral preparation or a preparation for external application;
preferably, the compound Nib1 in the oral preparation is administered at 0.1-1.098 mg/kg per time, once a day; and/or, the compound Nib1 in the preparation for external application is administered at 5-15 mg/cm² each time, at least once a day;
more preferably,
the preparation for external application comprises, by weight, 0.2-0.3 parts of Nib1 API, 0.3-0.5 parts of acetic acid, 5-6 parts of oil phase A, 4-5 parts of oil phase B, 1-2 parts of emulsifier A, 0.3-0.4 parts of emulsifier B, 2.5-3.5 parts of solvent A, 4-5 parts of solvent B, and 25-35 parts of water; alternatively, the preparation for external application comprises, by weight, 0.2-0.3 parts of Nib1 API, 5-6 parts of oil phase A, 4-5 parts of oil phase B, 1-2 parts of emulsifier A, 0.4-0.6 parts of emulsifier B, 2.5-3.5 parts of solvent A, 4-5 parts of solvent B, and 25-35 parts of water.

15. The medicament for treatment of psoriasis of claim 14, wherein the oil phase A is cetostearyl alcohol, the oil phase B is medium chain triglycerides, the emulsifier A is Tween 60, the emulsifier B is Span 80, the solvent A is dimethyl sulfoxide, and the solvent B is benzyl alcohol;
preferably, the preparation for external application is prepared by a method comprising steps of:
(1) weighing Nib1 and adding it in a container, then adding acetic acid to make the acetic acid just over the Nib1 API, and the whole white solid turns into transparent crystals, followed by adding a mixed solvents consisting of dimethyl sulfoxide and benzyl alcohol, stirring at room temperature to dissolve a part of the transparent crystal, and then placing it in a water bath at 65-75°C for 4-6 minutes to dissolve Nib1;
(2) adding medium-chain triglycerides to the solution obtained in step (1); adding cetostearyl alcohol and emulsifier, and placing in a water bath at 65-75°C for 10-20 minutes to obtain a transparent oil phase; and
(3) adding water at the same temperature to the transparent oil phase, taking it out and stirring until it cools to become a cream;
alternatively, the preparation for external application is prepared by a method comprising steps of:
(1) weighing Nib1 and adding it to a mixed solvent composed of dimethyl sulfoxide and benzyl alcohol, shaking and stirring, and then placing it in a water bath at 65-75°C for 15-25 minutes to obtain an oil phase solution;
(2) adding medium-chain triglycerides, cetostearyl alcohol, Tween 60 and Span 80 to the oil phase solution, and then heating and melting in a water bath at 65-75°C to obtain a transparent oil phase solution; and
(3) adding water at the same temperature to the transparent oil phase solution, stirring continuously, taking it out and stirring until it cools to become a cream.
